(19) Europäisches Patentamt
European Patent Office
Office européen des brevets

(11) **EP 1 442 291 B1**

(12) **FASCICULE DE BREVET EUROPEEN**

(45) Date de publication et mention
de la délivrance du brevet:
**18.02.2009 Bulletin 2009/08**

(21) Numéro de dépôt: **02793169.0**

(22) Date de dépôt: **18.10.2002**

(51) Int Cl.:
***G01N 33/48*** (2006.01)

(86) Numéro de dépôt international:
**PCT/FR2002/003572**

(87) Numéro de publication internationale:
**WO 2003/040719 (15.05.2003 Gazette 2003/20)**

(54) **MARQUEUR BIOLOGIQUE DES ETATS DE STRESS**

STRESS BIOLOGISCHE MARKER

BIOLOGICAL MARKER FOR STRESS STATES

(84) Etats contractants désignés:
**AT BE BG CH CY CZ DE DK EE ES FI FR GB GR
IE IT LI LU MC NL PT SE SK TR**

(30) Priorité: **06.11.2001 FR 0114310**

(43) Date de publication de la demande:
**04.08.2004 Bulletin 2004/32**

(73) Titulaire: **AR2I SA - Analyses, Recherches et
Innovation
Instrumentale
92350 Le Plessis Robinson (FR)**

(72) Inventeurs:
• **SARBACH, Christian
F-78000 Versailles (FR)**
• **DELVORDRE, Pascal
F-91300 Massy (FR)**
• **POSTAIRE, Eric
F-92170 Vanves (FR)**

(74) Mandataire: **Schwartz, Thierry J.
Cabinet ORES
36, rue de St Pétersbourg
75008 Paris (FR)**

(56) Documents cités:
**EP-A- 1 096 245**

**Description**

L'invention concerne un marqueur biologique (ou bio-marqueur) révélateur des états de stress et un procédé d'évaluation quantitative de la présence de ce marqueur biologique des états de stress.

La notion d'états de stress couvre l'ensemble des réactions métaboliques et/ou comportementales, provoquées dans l'organisme dans son entier par de nombreux facteurs d'agression exogènes, tels que : maladies inflammatoires, interventions chirurgicales, chocs traumatiques, rayonnements solaires, électromagnétiques ou ionisants, tabagisme, pollution, allergies, efforts prolongés, émotions, froid, etc.

Le type de perturbation engendrée dans l'organisme, permet de distinguer différents types ou états de stress : d'ordre chimique, microbiologique, biochimique, physiologique, psychique, biophysique, ou pharmacologique.

Il est bien établi que, sous l'effet de certains des facteurs cités, l'organisme produit des agents oxydants générant un stress dit oxydatif. Ces agents se présentent sous la forme d'espèces réactives de l'oxygène (ROS, initiales de « reactive oxygen species » en dénomination anglaise). Par exemple, en cas d'effort prolongé, la demande en oxygène, et par conséquent sa consommation, augmente ce qui induit à la fois un état d'hypoxie et une surproduction d'agents ROS.

La production des agents ROS est alors liée à des mécanismes biochimiques endogènes normaux. En particulier, dans la chaîne respiratoire, 2 à 4% de l'oxygène impliqué sont incomplètement réduits et donnent naissance à des agents ROS.

Les agents ROS sont des radicaux libres, des atomes ou molécules instables et réactives, présentant habituellement un ou plusieurs électrons célibataires. Leur origine est à la fois exogène et endogène au niveau des mitochondries. Les principaux agents ROS sont : l'oxygène singulet (O-), l'anion superoxyde, le peroxyde d'hydrogène, le radical hydroxyle, le monoxyde d'azote, ou les hydroperoxy radicaux (produits lors de la peroxydation des lipides membranaires, principalement ceux constitués d'acides gras polyinsaturés).

Sur le plan biochimique, le stress oxydatif conduit à :

- la peroxydation lipidique, ciblée sur les membranes cellulaires et mitochondriales ; les acides gras poly-insaturés (PUFA) sont attaqués et libèrent de l'éthane ($\omega$-3 PUFA) et du pentane ($\omega$-6 PUFA) ;
- l'oxydation protéique des protéines mitochondriales, qui conduit à des dysfonctionnements de la chaîne respiratoire et à une baisse de la production énergétique des cellules ; ou
- l'oxydation de l'ADN mitochondrial (ADNmt) qui conduit à des mutations entraînant également le dysfonctionnement des mitochondries.

Or l'oxydation est le principal responsable du vieillissement cellulaire et des maladies dues à l'âge (cancers, troubles cardiovasculaires, baisse des fonctions immunitaires, dysfonctionnement cérébral comme la maladie d'Alzheimer, ou la cataracte). Ceci est corroboré par le fait que les antioxydants alimentaires (acide ascorbique, tocophérol et caroténoïdes des fruits et végétaux) participent à la prévention contre l'apparition de ces maladies dégénératives.

L'éthane et le pentane constituent des bio-marqueurs connus des états de stress oxydatifs. Comme indiqué ci-dessus, le stress oxydatif provoque des désordres métaboliques dont la peroxydation lipidique qui induit la formation d'éthane et de pentane. Ces produits sont des substances volatiles qui sont ensuite éliminées dans l'air expiré.

Le pentane semble plus significatif que l'éthane *in vivo* car les lipides de structure $\omega$-6 PUFA sont prédominants au niveau membranaire, par rapport aux $\omega$-3 PUFA. La concentration mesurée en éthane/pentane dans l'air expiré est proportionnelle à l'état de stress oxydatif.

Cependant, il est important de disposer d'un marqueur quantitatif des états de stress, en particulier du stress oxydatif.

Le but de l'invention est donc de proposer un nouveau bio-marqueur des états de stress, qui soit hautement significatif et puisse permettre de mesurer quantitativement des niveaux de stress.

Pour ce faire, il a été détecté, de manière surprenante, que l'air de fin d'expiration d'une personne, dit air alvéolaire, contient des composants fluorés lorsque cette personne a subi différents états de stress.

Plus précisément, la présente invention a pour objet l'utilisation du trichloro-trifluoro-éthane comme biomarqueur des états de stress et un procédé pour évaluer un niveau de stress d'un état de stress chez un individu à partir d'un échantillon de fluide biologique, comprenant une étape de fractionnement et concentration de l'échantillon, et une étape d'évaluation quantitative de la présence de trichloro-trifluoro-éthane.

Des caractéristiques préférées se trouvent dans les revendications dépendantes.

D'autres caractéristiques et avantages apparaîtront à la lecture de la description détaillée qui suit, relative à des exemples de mise en oeuvre de l'invention pour différents états de stress en référence aux figures annexées, qui représentent respectivement :

- la figure 1, deux chromatogrammes d'air alvéolaire prélevé sur une personne, respectivement, au repos et après 15 minutes de marche rapide ;

- la figure 2, un chromatogramme d'air alvéolaire prélevé sur une personne, 5 minutes après un effort sportif intensif effectué pendant une heure ;
- la figure 3, un chromatogramme d'air alvéolaire prélevé sur la même personne, 6 heures après cet effort sportif intensif ;
- la figure 4, deux chromatogrammes d'air alvéolaire prélevé sur un fumeur occasionnel, respectivement, au repos et 5 minutes après avoir fumé ; et
- la figure 5, deux chromatogrammes superposés d'air alvéolaire prélevé, respectivement, sur un fumeur occasionnel 5 minutes après qu'il a fumé, et sur un fumeur chronique au repos.

Le dispositif de prélèvement d'air expiré utilisé pour effectuer les mesures exposées ci-après est du type décrit dans la demande de brevet publiée sous le numéro FR 2 800 465, déposée au nom de la demanderesse et incorporée par référence. Ce dispositif permet de prélever et de concentrer spécifiquement l'air de fin d'expiration, dit air alvéolaire, qui représente une fraction riche en bio-marqueurs présents dans l'air pulmonaire, avec une haute sensibilité et une haute reproductibilité du prélèvement. Des protocoles communs, concernant les conditions mise sous stress et de prélèvement d'air, ont été respectés dans les différents exemples ci-après exposés, afin de vérifier la reproductibilité des résultats.

Les prélèvements se présentent sous forme de cartouche remplie de deux couches d'adsorbant. Les analyses des différents prélèvements sont réalisées par désorption thermique, suivie d'une chromatographie en phase gazeuse avec détection par spectrométrie de masse.

La famille chimique des chloroalcanes fluorés, encore appelée CFC (initiales de Chloro-Fluoro-Carbones) ou fréons, ont ainsi été mesurés comme bio-marqueurs du stress dans l'air alvéolaire expiré. Dans les exemples qui suivent, le tri-chloro-trifluoro-éthane $C_2Cl_3F_3$, est plus particulièrement mis en évidence.

La structure chimique de ce bio-marqueur a été déterminée par la spectrométrie de masse couplée à la chromatographie gazeuse.

Les exemples d'augmentation de la production et de l'élimination du bio-marqueur selon l'invention qui sont illustrés, sont provoqués par différents types de stress.

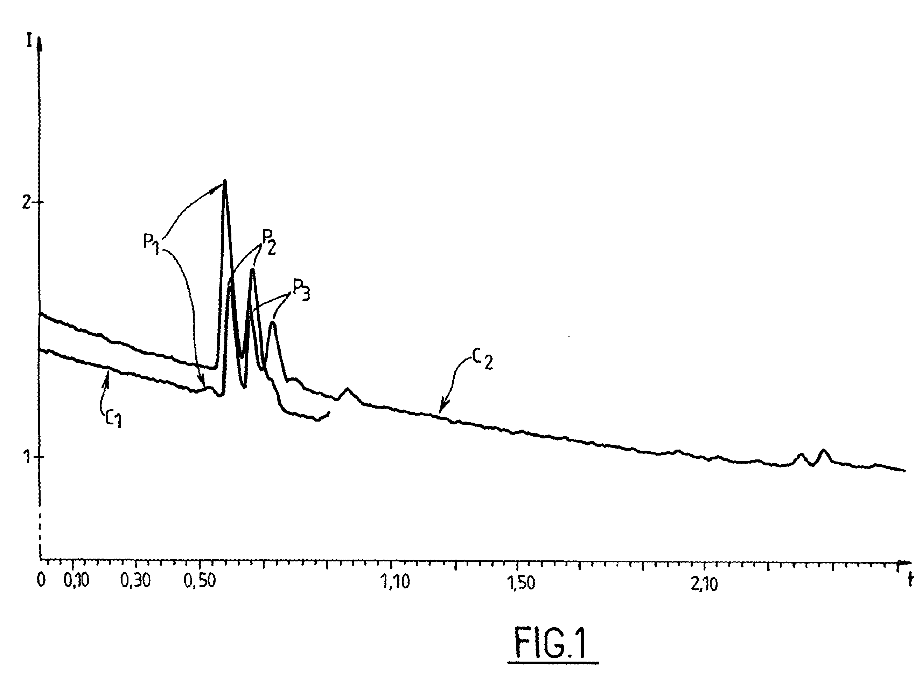

En référence à la figure 1, un effort modéré est provoqué pour illustrer le stress physiologique. Les deux chromatogrammes représentés, C1 et C2, correspondent à l'analyse chromatographique de l'air alvéolaire prélevé sur une personne, respectivement, au repos (courbe C1) et après 15 minutes de marche rapide (courbe partielle C2). L'intensité I de signal est représentée en fonction du temps t. Les unités sont arbitraires. Les deux chromatogrammes sont légèrement décalés pour améliorer la visibilité de la figure.

Les pics P1 correspondent à la présence de $C_2Cl_3F_3$, les pics P2 à la présence d'isoprène, et les pics P3 à celle de dichlorométhane. La différence d'intensité des pics P1 illustre bien la sensibilité du $C_2Cl_3F_3$ en tant que bio-marqueur du stress de type physiologique.

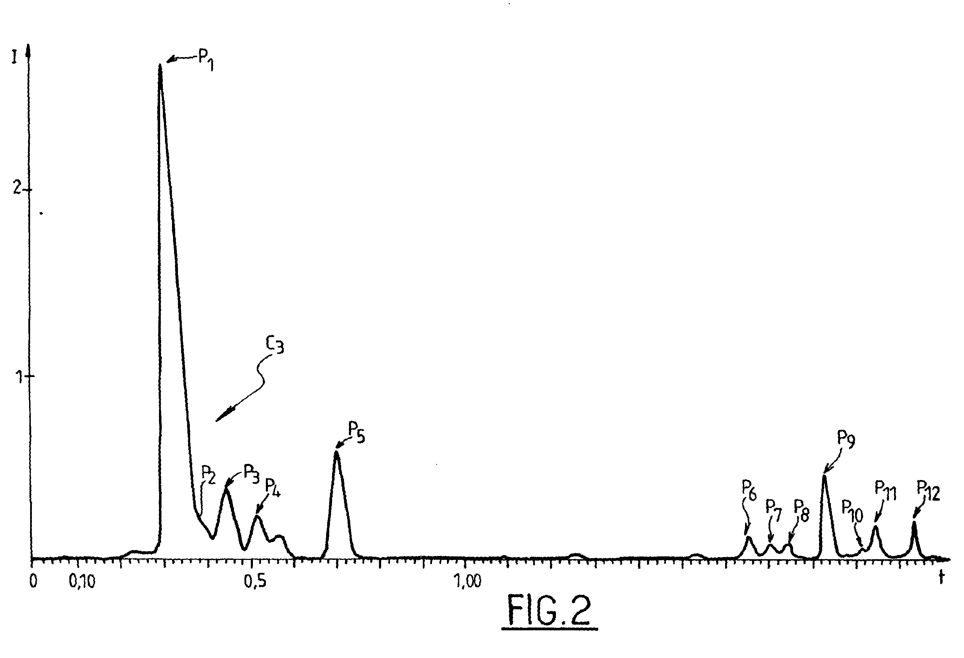

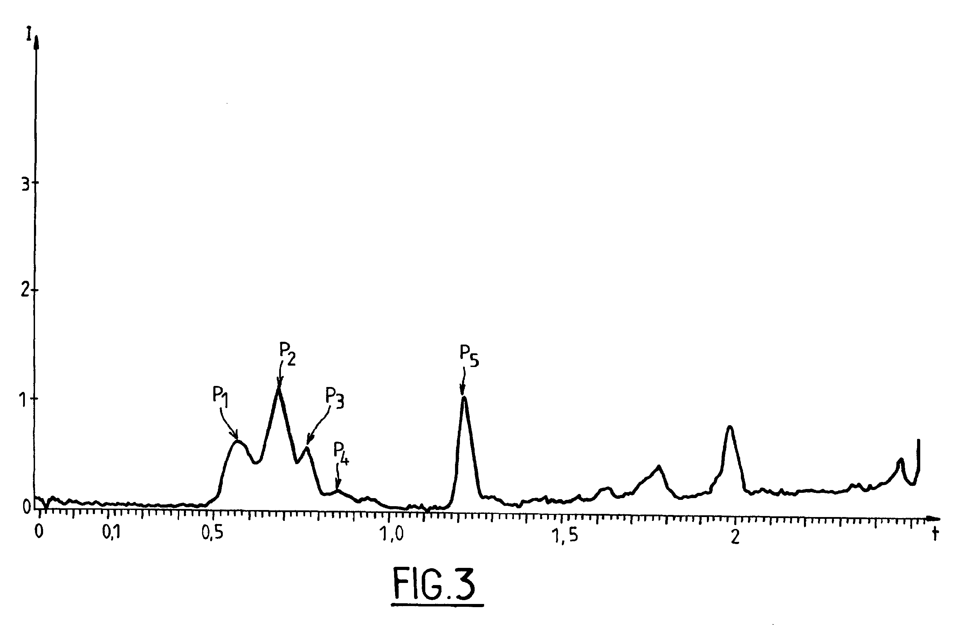

Les figures 2 et 3 illustrent le cas d'un effort avec essoufflement intense de la personne, provoqué par la pratique du tennis pendant une heure, toujours pour illustrer le stress physiologique.

Sur la figure 2, le chromatogramme C3 d'air alvéolaire est prélevé sur la personne, 5 minutes après l'effort sportif. L'amplitude élevée du pic P1 traduit le niveau de stress subi par l'organisme du fumeur. Les pics P4 à P12 correspondent, respectivement, au cyclohexane (P4), à l'hexane (P5), au chloroforme (P6), au méthylcyclohexane (P7), au triméthylhexane (P8), à l'heptane (P9), au tétrachloroéthylène (P10), au benzène (P11) et à l'éthyléther (P12).

Sur la figure 3, soit 6 heures après l'effort sportif intensif, le pic P1 du chromatogramme C4 est sensiblement diminué, ce qui illustre encore la fonction de bio-marqueur de stress dédiée au trichlorotrifluoroéthane $C_2Cl_3F_3$.

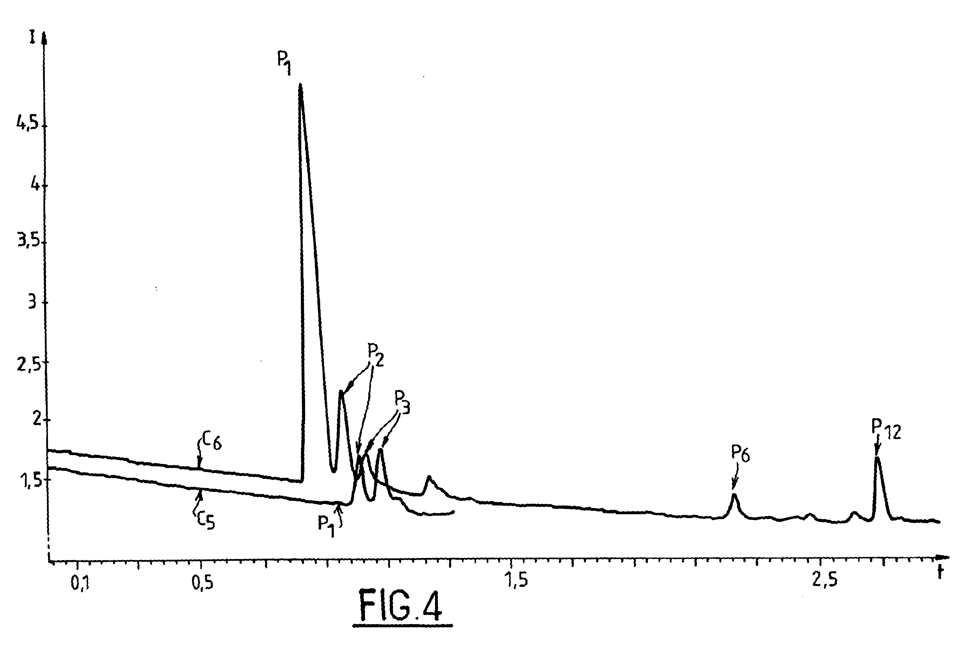

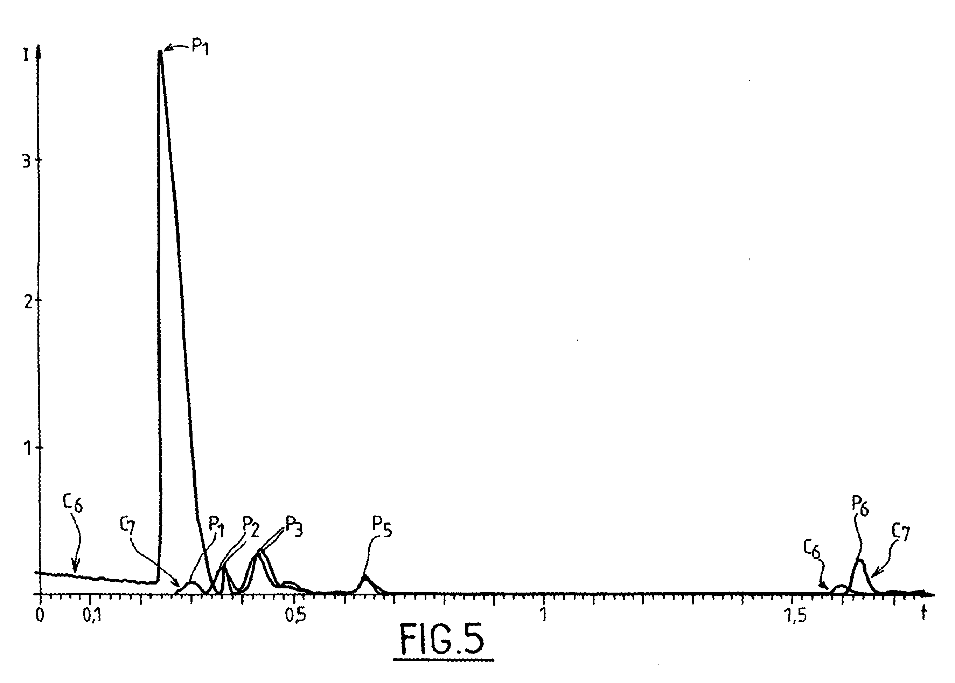

Les figures 4 et 5 illustrent également la sensibilité du $C_2Cl_3F_3$ comme révélateur du stress de type chimique.

Sur la figure 4, les deux chromatogrammes, C5 (reporté partiellement) et C6, représentent l'air alvéolaire prélevé sur un fumeur occasionnel, respectivement, au repos et 5 minutes après avoir fumé.

Les pics P1 correspondent encore à la présence de $C_2Cl_3F_3$, les pics P2 à la présence d'isoprène, et les pics P3 à celle de dichlorométhane. D'autres pics sont identifiés : le pic P6 correspond au chloroforme et le pic P12 à l'éthyléther.

Sur la figure 5, les deux chromatogrammes, courbes C6 et C7 superposées, représentent l'air alvéolaire prélevé, respectivement, sur le fumeur occasionnel 5 minutes après avoir fumé comme sur la figure précédente, et, pour comparaison, sur un fumeur chronique au repos, n'ayant pas fumé au moins pendant l'heure qui a précédé le prélèvement. Sur cette figure, les pics P5 (hexane) et P6 (chloroforme) de l'heptane ont également été indiqués.

La comparaison des pics P1 sur les deux chromogrammes montre bien que le bio-marqueur selon l'invention, le $C_2Cl_3F_3$, est bien révélateur d'un état de stress passager et non d'un état chronique. Un étalonnage de la quantité de $C_2Cl_3F_3$ est effectué par rapport au dichlorométhane, présent dans l'air alvéolaire et qui varie peu, sous la forme du rapport F suivant, entre les intensités des signaux des pics de chromatographie :

$$F = \frac{\text{signal du pic chromatographique du biomarqueur}}{\text{signal du pic chromatographique du dichlorométhane}}$$

Un tel étalonnage F est rapporté dans le tableau ci-après, pour différents exemples d'états de stress pris à partir d'un état de repos et en général 5 minutes après l'arrêt (sauf indication contraire) de diverses causes du stress, à savoir : le stress physiologique par la pratique de sports (violent et modéré), le stress biochimique dû au tabagisme, le stress psychique par la pratique intense d'un jeu vidéo rapide, et le stress biophysique par une exposition aux rayonnements électromagnétiques pulsés d'un écran d'ordinateur.

Les mesures étalonnées F du bio-marqueur, prises au repos ($F_0$) et après soumission aux différents stress dans les conditions indiquées, sont résumées dans le tableau suivant :

| TYPE DE STRESS | $F_0$ | CONDITION DE SOUMISSION | F |
|---|---|---|---|
| Physique modéré | 0,37 | 20 minutes de vélo d'appartement | 0,48 |
| Physique modéré | 0,13 | 15 minutes de marche à pied rapide | 3,14 |
| Physique violent | 0,21 | 1 heure de tennis<br>6 heures après l'effort | 7,87<br>0,59 |
| biochimique | 0,13 | Consommation d'une cigarette | 12,5 |
| psychique | 0,37 | 30 minutes de pratique intense d'un jeu vidéo rapide | 5,00 |
| biophysique | 0,21 | 30 minutes d'exposition au rayonnement électromagnétique d'un écran d'ordinateur<br>15 minutes après l'exposition<br>90 minutes après l'exposition | 0,88<br><br>0,44<br>0,21 |

Ainsi, quel que soit le type de stress, il est possible de quantifier, à partir des valeurs obtenues, des niveaux de stress pour chaque type de stress.

**Revendications**

1. Utilisation **du trichloro-trifluoro-éthane** comme bio-marqueur des états de stress.

2. Utilisation selon **la revendication 1,** dans laquelle le **trichloro-trifluoro-éthane** est obtenu à partir d'un échantillon d'un fluide biologique produit par le corps humain, par fractionnement et concentration de l'échantillon.

3. Utilisation selon la revendication **2,** dans laquelle l'échantillon sur lequel sont effectués le fractionnement et la concentration, est une portion finale d'un prélèvement de fluide.

4. Utilisation selon les revendications **2** ou **3,** dans laquelle le **trichloro-trifluoro-éthane** est obtenu à partir d'air alvéolaire.

5. Procédé pour évaluer un niveau de stress d'un état de stress chez un individu à partir d'un échantillon de fluide biologique, comprenant une étape de fractionnement et concentration de l'échantillon, et une étape d'évaluation quantitative de la présence **de trichloro-trifluoro-éthane.**

6. Procédé selon la revendication **5,** dans lequel l'étape d'évaluation quantitative est réalisée par chromatographie en phase gazeuse couplée à une détection par spectrométrie de masse.

7. Procédé selon la revendication **6,** dans lequel une détermination quantitative d'un niveau de stress pour chaque état de stress est ensuite effectuée par étalonnage.

**Claims**

1. Use of the trichloro-trifluro-ethane as a biomarker for stress states.

2. The use according to claim 1, wherein the trichloro-trifluro-ethane is obtained from a sample of a biological fluid produced by the human body, by fractioning and concentrating of said sample.

3. The use according to claim 2, wherein the sample on which are performed the fractioning and the concentrating is a final portion of sample of said fluid.

4. The uses according to claims 2 or 3, wherein the trichloro-trifluro-ethane is obtained from an alveolar air.

5. A method to evaluate the stress level of a stress state at in individual from a sample of biological fluid comprising a step of fractioning and concentrating of said sample, and a step of quantitatively evaluating the presence of the trichloro-trifluro-ethane.

6. The method according to claim 5, wherein the step of quantitatively evaluating is performed by gas chromatography coupled with mass spectrometry detection.

7. The method according to claim 6, wherein a quantitative determination of a stress level for each stress state is subsequently performed by calibration.


**Patentansprüche**

1. Verwendung von Trichlortrifluorethan als Biomarker für Stresszustände.

2. Verwendung gemäß Anspruch 1, bei der Trichlortrifluorethan aus einer Probe eines biologischen Fluids, das durch den menschlichen Körper produziert wird, durch Fraktionierung und Konzentrierung der Probe erhalten wird.

3. Verwendung gemäß Anspruch 2, bei der die Probe, an der die Fraktionierung und die Konzentrierung durchgeführt werden, eine Endportion einer Fluidentnahme ist.

4. Verwendung gemäß Anspruch 2 oder 3, bei der Trichlortrifluorethan aus Alveolarluft erhalten wird.

5. Verfahren zur Evaluierung eines Stresslevels eines Stresszustandes bei einem Individuum aus einer Probe von biologischem Fluid, umfassend eine Stufe der Fraktionierung und Konzentrierung der Probe und eine Stufe der quantitativen Evaluierung des Vorliegens von Trichlortrifluorethan.

6. Verfahren gemäß Anspruch 5, bei dem die Stufe der quantitativen Evaluierung durch Gaschromatographie, gekoppelt an eine Detektion durch Massenspektrometrie, durchgeführt wird.

7. Verfahren gemäß Anspruch 6, bei dem eine quantitative Bestimmung eines Stresslevels für jeden Stresszustand dann durch Kalibrierung durchgeführt wird.

EP 1 442 291 B1

FIG.1

FIG.2

EP 1 442 291 B1

FIG.3

EP 1 442 291 B1

FIG.4

EP 1 442 291 B1

EP 1 442 291 B1

FIG.5

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2800465